# EUROPÄISCHE PATENTSCHRIFT

(11) **EP 1 402 831 B1**
(45) Veröffentlichungstag und Bekanntmachung des Hinweises auf die Patenterteilung: **03.12.2008**
(21) Anmeldenummer: 03014896.9
(22) Anmeldetag: 01.07.2003
(51) Int. Cl.: A61B 17/72

(54) **Humerusnagel**
Humerus nail
Clou huméral

(30) Priorität: 28.08.2002 DE 20213166 U
(43) Veröffentlichungstag der Anmeldung: 31.03.2004
(73) Patentinhaber: Stryker Trauma GmbH, 24232 Schönkirchen (DE)
(72) Erfinder: Ekholm, Carl, 43931 Onsala (SE); Jönsson, Anders, 43994 Onsala (SE); Zander, Nils, 24340 Eckernförde (DE)
(74) Vertreter: Maiwald Patentanwalts GmbH

(56) Entgegenhaltungen:
- EP-A- 0 715 832
- EP-A- 0 853 923
- WO-A-99/35989
- DE-A1- 19 945 611
- US-A- 5 472 444

## Beschreibung

Die Erfindung bezieht sich auf einen Humerusnagel nach dem Oberbegriff des Anspruchs 1.

Es ist bekannt, dass Frakturen des Oberarms häufig im proximalen Bereich, insbesondere im Kopfbereich des Humerus auftreten. Zur Versorgung derartiger Frakturen ist bereits bekannt, einen so genannten Verriegelungsnagel vorzusehen. Er wird von proximal in den Humeruskanal eingetrieben und weist sowohl im proximalen als auch im distalen Bereich Verriegelungsbohrungen auf, durch welche Knochenschrauben hindurch geführt werden, um den Verriegelungsnagel gegen axiale Verschiebung und Torsion zu sichern. Die Knochenschrauben im proximalen Bereich dienen außerdem zur Fixierung der Knochenfragmente.

Ein Humerusnagel der beschriebenen Art ist etwa aus US 5 427 444 bekannt geworden. Der Nagel weist einen länglichen Schaft auf und hat im proximalen Bereich vier Querbohrungen, die axial beabstandet und in Umfangsrichtung jeweils gegeneinander versetzt angeordnet sind. Der Nagelschaft ist gekrümmt.

Der Erfindung liegt die Aufgabe zugrunde, einen Humerusnagel der genannten Art dahingehend zu verbessern, dass eine noch wirksamere Fixierung des Nagels bzw. der Frakturfragmente erfolgen kann.

Diese Aufgabe wird mit den Merkmalen des Anspruchs 1 gelöst.

Bei dem erfindungsgemäßen Humerusnagel ist die proximale Querbohrung, d.h. diejenige, die dem proximalen Ende am nächsten liegt, mit ihrer Achse schräg zur Längsachse des Nagelschafts angeordnet.

Bei einem proximal in den Humerus eingeführten Nagel liegt der Nagel auf der Außenseite des Humeruskopfes, und die Querbohrungen im proximalen Abschnitt sind so angeordnet, dass die Verriegelungsschrauben unter verschiedenen Richtungen in den Humeruskopf eingeschraubt werden können. Bei dem erfindungsgemäßen Nagel ist die schräge Anordnung der proximalen Querbohrung derart, dass die Knochenschraube von außen nach innen schräg nach unten eingeschraubt wird. Dadurch ist die Knochenschraube anatomisch günstiger angeordnet, weil dadurch die Kraftübertragung auf den Nagel günstiger gestaltet ist. Außerdem kann die Knochenschraube eine größere Länge erhalten, da sie über eine größere Länge in den Humeruskopf eingebracht werden kann. Insgesamt ist daher die Versorgung von Frakturen im Kopfbereich des Humerus durch den erfmdungsgemäßen Nagel verbessert.

Nach einer Ausgestaltung der Erfindung ist die Neigung der Achse der proximalen Querbohrung zur Längsachse des Nagelschaftes in einem Winkel von etwa 80°.

Wie schon erwähnt, sind im proximalen Abschnitt des Nagelschaftes mehrere Querbohrungen vorgesehen. Nach einer Ausgestaltung der Erfindung ist die Achse der distalen Querbohrung im proximalen Abschnitt, d.h. mithin der Querbohrung, die den größten Abstand zum proximalen Ende des Nagelschaftes hat, ebenfalls schräg zur Längsachse des Nagelschaftes angeordnet. Die Schrägung ist derart, dass die Achsen von proximaler und distaler Querbohrung konvergieren bzw. divergieren. Sie sind mithin entgegengesetzt gerichtet. Auch diese Maßnahme erweist sich bei Frakturen des Humeruskopfes als äußerst vorteilhaft, da auch hier die Knochenschraube länger gewählt und wirksamer in die gefährdeten Bereiche des Humeruskopfes eingebracht werden kann.

Nach einer Ausgestaltung der Erfindung ist der Winkelversatz der distalen Querbohrung gegenüber der proximalen Querbohrung annähernd 25°, wobei dieser Versatz vorzugsweise entgegengesetzt gerichtet ist zum Versatz derjenigen Querbohrung, welche der proximalen Querbohrung folgt.

Vorzugsweise sind im proximalen Abschnitt vier Querbohrungen vorgesehen, wobei vorzugsweise die beiden mittleren Bohrungen mit ihrer Achse senkrecht zur Längsachse des Nagelschaftes verlaufen.

Nach einer anderen Ausgestaltung der Erfindung kann der Nagelschaft massiv ausgebildet sein. Nach einer weiteren Ausgestaltung der Erfindung ist eine der beiden distalen Querbohrungen im distalen Bereich als achsparalleles Langloch ausgeführt.

Bei gerade verlaufendem Schaft ist es zweckmäßig, wenn für den linken und den rechten Humerus zwei getrennte Humerusnägel vorgesehen sind. Nach einer Ausgestaltung der Erfindung sind mit Ausnahme der proximalen Querbohrung die übrigen Querbohrungen in einer unterschiedlichen Anordnung für den linken und rechten Nagelschaft vorgesehen, wobei die beiden Anordnungen im Hinblick auf den Abstand und die Winkellage sich zwar gleichen, jedoch um 180° gedreht sind, bezogen auf die Längsachse.

Ein Ausführungsbeispiel der Erfindung soll nachfolgend anhand von Zeichnungen näher erläutert werden.

Fig. 1 zeigt die Längsansicht eines Humerusnagels für den rechten Humerus in Seitenansicht.

Fig. 2 zeigt die Seitenansicht des Nagels nach Fig. 1 um 90° verdreht.

Fig. 3 zeigt einen Schnitt durch den Nagel nach Fig. 1 entlang der Linie 3-3.

Fig. 4 zeigt die Seitenansicht eines erfindungsgemäßen Humerusnagels für den linken Humerus in einer Ansicht entsprechend Fig. 2.

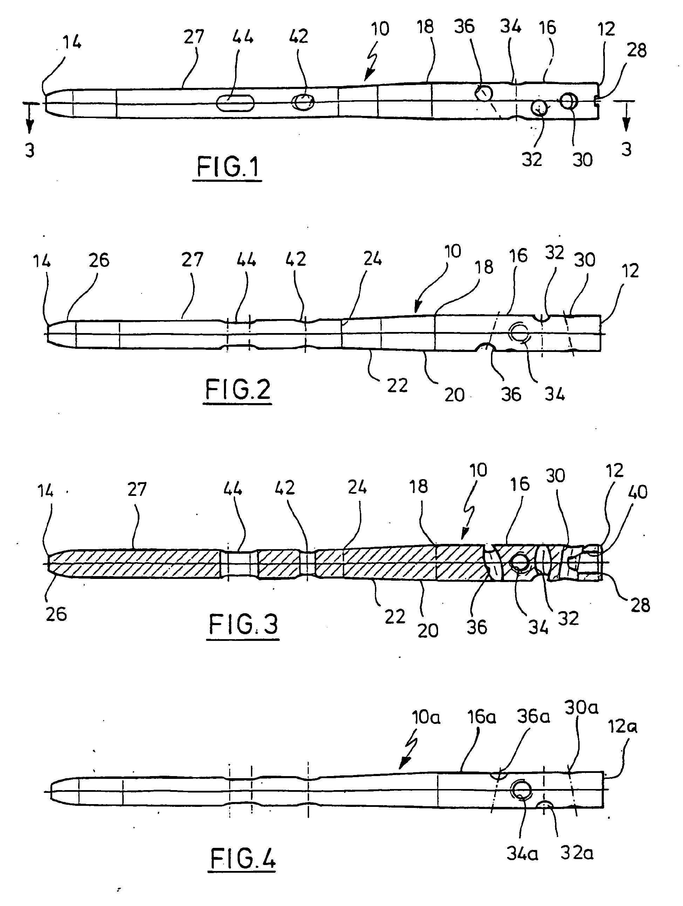

In den Figuren 1 bis 3 ist ein Humerusnagel 10 für den rechten Humerus dargestellt. Er weist einen geraden Schaft auf mit einem proximalen Ende 12 und einem distalen Ende 14. Der Nagel 10 wird von proximal in den Humerus eingeführt und dient zur Versorgung von Frakturen im proximalen Bereich des Humerus (nicht gezeigt). Der Nagel 10 ist als Verriegelungsnagel ausgeführt.

Der Nagelschaft weist einen proximalen Abschnitt 16 auf, der sich bis zu einer Kante 18 erstreckt. Der proximale Abschnitt 16 hat konstanten Durchmesser. Ab der Kante 18, die auch als relativ weicher Übergang geformt sein kann, folgt ein relativ kurzer konischer Abschnitt 20 und diesem ein weiterer konischer Abschnitt 22. Von der Kante 24 des konischen Abschnitts 22 erstreckt sich der Nagelschaft bis zum distalen Ende 14 annähernd mit gleichem geringeren Durchmesser, wobei der distale Endabschnitt 26 wiederum konisch ausgebildet ist oder kugelförmig sein kann.

Das proximale Ende 22 ist mit drei senkrecht zur Längsachse des Nagelschaftes verlaufenden Querschlitzen 28 versehen, die mit entsprechenden Vorsprüngen in einem nicht dargestellten Ziel- und Einschlaginstrument zusammenwirken, damit der Nagel 10 in der richtigen Drehlage vom Instrument aufgenommen werden kann.

Im proximalen Abschnitt 16 befinden sich vier Querbohrungen 30, 32, 34 und 36. Die Achsen der mittleren Bohrungen 32, 34 stehen senkrecht auf der Längsachse des Nagels 10, sind jedoch um 115° zueinander verdreht. Die Achse der proximalen Querbohrung 30 ist schräg zur Längsachse angeordnet, z.B. in einem Winkel von annähernd 80°. Die proximale Querbohrung 30 schneidet teilweise eine axiale Gewindebohrung 40 des Nagels 10, die zur Verbindung mit dem nicht gezeigten Einschlag- und Zielinstrument dient.

Die der proximalen Bohrung 30 nächstfolgende Bohrung 32 ist am Umfang um einen bestimmten Winkel versetzt angeordnet von z.B. 25°. Dieser Versatz ist relativ gut in Fig. 1 zu erkennen. Die distale Querbohrung 36 verläuft mit ihrer Achse ebenfalls schräg zur Längsachse des Nagels 10, z.B. einem Winkel von 75°, wobei die Achsen der Querbohrungen 30, 36 konvergieren bzw. divergieren. Die Achse der distalen Querbohrung 36 ist außerdem gegenüber der proximalen Querbohrung 30 in Umfangsrichtung verschwenkt, wiederum um annähernd 25°, wobei jedoch die Verschwenkung gegenüber der Querbohrung 32 in der entgegengesetzten Richtung erfolgt, wie sich wiederum aus Fig. 1 ergibt.

Die Querbohrungen 30 bis 36 dienen zur Aufnahme von Knochenschrauben (nicht gezeigt), die durch die Kortikalis des Humerus in den Humeruskopf eingeschraubt werden. Zum Auffinden der Querbohrungen 30 bis 36 ist ein entsprechendes Zielgerät (nicht gezeigt) erforderlich. Die Versetzung der Querbohrungen in Umfangsrichtung zueinander ermöglicht das Ansetzen der Knochenschrauben aus unterschiedlichen Richtungen, um die jeweiligen Frakturen im Humeruskopf optimal zu versorgen. Die schräge Anordnung der Querbohrungen 30 und 36 ermöglicht eine noch optimalere Frakturversorgung bei besserer Kraftübertragung vom Knochen auf den Nagel und umgekehrt. Außerdem ermöglicht die schräge Anordnung der Querbohrungen 30 und 36 die Verwendung von besonders langen Knochenschrauben, ohne dass die Gefahr besteht, dass der Humeruskopf durchstoßen wird. Die Bohrungen 32 bis 36 weisen ein Gewinde auf, das mit den Verriegelungs- oder Knochenschrauben korrespondiert. Damit wird ein unbeabsichtigtes Auswandern der Schrauben verhindert.

Im Abschnitt 27 des Nagels 10 sind relativ im Abstand zum distalen Ende 14 zwei Querbohrungen 42, 44 vorgesehen, deren Achsen in einer Ebene liegt, in der auch die Achse der proximalen Querbohrung 30 liegt. Die distal gelegene distale Querbohrung 44 ist als Langloch ausgeführt, was insbesondere aus den Figuren 1 und 3 zu erkennen ist. Die Querbohrungen 42, 44 dienen wiederum zur Aufnahme von Knochenschrauben zur Verankerung des Nagels 10 im Humerusschaft.

Wie schon erwähnt, dient der Nagel 10 nach den Figuren 1 bis 3 zur Versorgung von Frakturen im rechten Humerus. Ein Nagel 10a, wie er in Fig. 4 dargestellt ist, ist mit seinem Nagelschaft identisch zu dem Nagel 10 nach den Figuren 1 bis 3 ausgebildet. Daher soll auf die Einzelheiten des Nagels 10a nicht weitere eingegangen werden, mit Ausnahme des proximalen Abschnitts 16a, der ebenfalls vier Querbohrungen 30a, 32a, 34a und 36a aufweist. Die Ausbildung der Querbohrungen 30a bis 36a gleicht wiederum der nach den Figuren 1 bis 3. Auch die Lage der proximalen Querbohrung 30a ist identisch zu der Querbohrung 30 nach den Figuren 1 bis 3. Lediglich die Anordnung der Querbohrungen 32a bis 36a ist unterschiedlich zu der nach den Figuren 1 bis 3, indem diese Anordnung gegenüber der z.B. nach Fig. 2 gespiegelt ist. Die unterschiedliche Anordnung beim Nagel 10a ergibt sich ausschließlich daraus, dass der Nagel 10a für den linken Humerus eingesetzt wird. Bezogen auf den anzuwendenden Humerus, d.h. links oder rechts, ist daher die Anordnung der Querbohrungen 30 bis 36 bzw. 30a bis 36a gleich.

## Patentansprüche

1. Humerusnagel für die Versorgung von Frakturen des proximalen Humerus, mit einem länglichen Schaft, der eine Längsachse hat und in einem proximalen Abschnitt (16, 16a) mindestens drei in axialer Richtung beabstandete Querbohrungen (30, 32, 34, 36, 30a, 32a 34a, 36a) aufweist, deren Achsen in Umfangsrichtung des Schaftes gesehen zueinander versetzt sind und der in einem zum distalen Ende hin liegenden Abschnitt mindestens zwei weitere Querbohrungen (42, 44) aufweist, wobei die dem proximalen Ende am nächsten liegende Querbohrung (30, 30a) in der gleichen Ebene liegt wie die Achse der weiteren Querbohrungen (42, 44), **dadurch gekennzeichnet, dass** die dem proximalen Ende am nächsten liegende Querbohrung (30, 30a) schräg zur Längsachse verläuft.

2. Humerusnagel nach Anspruch 1, **dadurch gekennzeichnet, dass** die Achse der proximalen Querbohrung (30) mit der Längsachse einen Winkel von etwa 80 DEG einschliesst.

3. Humerusnagel nach Anspruch 1 oder 2, **dadurch gekennzeichnet, dass** die Achse der distalen Querbohrung (36, 36a) im proximalen Abschnitt (16, 16a) ebenfalls schräg zur Längsachse verläuft derart, dass die Achsen von proximaler und distaler Querbohrung (30, 30a bzw. 36, 36a) konvergieren.

4. Humerusnagel nach Anspruch 3, **dadurch gekennzeichnet, dass** die Achse der distalen Querbohrung (36, 36a) zur Längsachse einen Winkel von annähernd 75 DEG einschliesst.

5. Humerusnagel nach Anspruch 4, **dadurch gekennzeichnet, dass** der Winkelversatz der distalen Querbohrung (36, 36a) gegenüber der proximalen Querbohrung (30, 30a) annähernd 25 DEG beträgt.

6. Humerusnagel nach einem der Ansprüche 3 bis 5, **dadurch gekennzeichnet, dass** im proximalen Abschnitt (16, 16a) des Nagelschaftes vier Querbohrungen (30 bis 36 bzw. 30a bis 36a) vorgesehen sind.

7. Humerusnagel nach Anspruch 6, **dadurch gekennzeichnet, dass** die der proximalen Querbohrung (30, 30a) folgende zweite Querbohrung (32, 32a) mit ihrer Achse auf der Längsachse annähernd senkrecht steht.

8. Humerusnagel nach Anspruch 6, **dadurch gekennzeichnet, dass** die der proximalen Querbohrung (30, 30a) folgende dritte Querbohrung (34, 34a) mit ihrer Achse annähernd auf der Längsachse senkrecht steht.

9. Humerusnagel nach einem der Ansprüche 6 bis 8, **dadurch gekennzeichnet, dass** der Winkelversatz von zweiter Querbohrung (32, 32a) zur proximalen Querbohrung (30, 30a) annähernd 25 DEG beträgt.

10. Humerusnagel nach einem der Ansprüche 6 bis 9, **dadurch gekennzeichnet, dass** der Winkelversatz von dritter Querbohrung (34) zur proximalen Querbohrung (30) annähernd 90 DEG beträgt.

11. Humerusnagel nach Anspruch 5 und 9, **dadurch gekennzeichnet, dass** der Winkelversatz von zweiter und vierter Querbohrung (32, 32a, 36, 36a) bezogen auf die Achse der proximalen Querbohrung (30, 30a) entgegengesetzt gerichtet ist.

12. Humerusnagel nach einem der Ansprüche 1 bis 11, **dadurch gekennzeichnet, dass** der Nagelschaft massiv ausgebildet ist.

13. Humerusnagel nach einem der Ansprüche 1 bis 12, **dadurch gekennzeichnet, dass** die distale Querbohrung (44) im distalen Bereich als achsparalleles Langloch geformt ist.

14. Humerusnagel nach einem der Ansprüche 1 bis 12, **dadurch gekennzeichnet, dass** ein Nagelschaft für den rechten und ein Nagelschaft für den linken Humerus vorgesehen ist und die Schäfte gerade ausgebildet sind, die Querbohrungen im proximalen Abschnitt für den linken und rechten Humerus unterschiedlich angeordnet sind derart, dass mit Ausnahme der proximalen Querbohrung (30, 30a) die Anordnung der Querbohrungen (32 bis 36 bzw. 32a bis 36a) für den linken Nagelschaft gegenüber der für die Querbohrungen des rechten Nagelschaftes gespiegelt ist.

15. Humerusnagel nach einem der Ansprüche 1 bis 14, **dadurch gekennzeichnet, dass** die Querbohrungen ein Gewinde aufweisen, das mit dem Gewinde von Verriegelungsschrauben korrespondiert.

## Claims

1. Humerus nail for the treatment of fractures of the proximal humerus, with a longitudinal shaft having a longitudinal axis, and comprising in a proximal portion (16, 16a) at least three axially spaced transverse bores (30, 32, 34, 36, 30a, 32a, 34a, 36a), the axes of which are offset to each other when viewed in the circumferential direction of the shaft and comprising in a portion lying toward the distal end at least two further transverse bores (42, 44), wherein the transverse bore lying closest to the proximal end (30, 30a) lies in the same plane as the axis of the further transverse bores (42, 44) **characterised in that** the transverse bore (30, 30a) lying closest to the proximal end extends inclined to the longitudinal axis.

2. Humerus nail according to claim 1, **characterised in that** the axis of the proximal transverse bore (30) encloses an angle of approximately 80° with the longitudinal axis.

3. Humerus nail according to claim 1 or 2, **characterised in that** the axis of the distal transverse bore (36, 36a) in the proximal portion (16, 16a) also extends inclined to the longitudinal axis is such a way that the axes of the proximal and distal transverse bores (30, 30a or 36, 36a) converge.

4. Humerus nail according to claim 3, **characterised in that** the axis of the distal transverse bore (36, 36a) encloses an angle of approximately 75° to the longitudinal axis.

5. Humerus nail according to claim 4, **characterised in that** the angular offset of the distal transverse bore (36, 36a) with respect to the proximal transverse bore (30, 30a) is approximately 25°.

6. Humerus nail according to any one of claims 3 to 5, **characterised in that** four transverse bores (30 to 36 or 30a to 36a) are provided in the proximal portion (16, 16a) of the nail shaft.

7. Humerus nail according to claim 6, **characterised in that** the axis of the second transverse bore (32, 32a) following the proximal transverse bore (30, 30a) stands approximately perpendicularly on the longitudinal axis.

8. Humerus nail according to claim 6, **characterised in that** axis of the third transverse bore (34, 34a) following the proximal transverse bore (30, 30a) stands approximately perpendicularly on the longitudinal axis.

9. Humerus nail according to any one of claims 6 to 8, **characterised in that** the angular offset of the second transverse bore (32, 32a) to the proximal transverse bore (30, 30a) is approximately 25°.

10. Humerus nail according to any one of claims 6 to 9, **characterised in that** the angular offset of the third transverse bore (34) to the proximal transverse bore (30) is approximately 90°.

11. Humerus nail according to claim 5 and 9, **characterised in that** the angular offset of the second and fourth transverse bore (32, 32a, 36, 36a) is directed opposite with respect to the axis of the proximal transverse bore (30, 30a).

12. Humerus nail according to any one of claims I to 11, **characterised in that** the nail shaft is solid.

13. Humerus nail according to any one of claims 1 to 12, **characterised in that** the distal transverse bore (44) in the distal region is shaped as an axially parallel slot hole.

14. Humerus nail according to any one of claims 1 to 12, **characterised in that** one nail shaft is provided for the right humerus and one nail shaft is provided for the left humerus and the shafts are straight, the transverse bores in the proximal portion for the left and right humerus are arranged differently in such a way that, apart from the proximal transverse bore (30, 30a), the arrangement of the transverse bores (32 to 36 or 32a to 36a) for the left nail shaft is a mirror image of that for the transverse bores of the right nail shaft.

15. Humerus nail according to any one of claims 1 to 14, **characterised in that** the transverse bores comprise a thread corresponding to the thread of locking screws.

## Revendications

1. Clou huméral pour soigner des fractures de l'humérus proximal, comprenant une tige allongée qui présente un axe longitudinal et, dans une section proximale (16, 16a), au moins trois trous transversaux (30, 32, 34, 36, 30a, 32a, 34a, 36a) distants dans la direction axiale, dont les axes sont en déport mutuel, vu dans la direction périphérique de la tige, et qui présente au moins deux autres trous transversaux (42, 44) dans une section située en direction de l'extrémité distale, le trou transversal (30, 30a) le plus proche de l'extrémité proximale se situant dans le même plan que l'axe des autres trous transversaux (42, 44), **caractérisé en ce que** le trou transversal (30, 30a) le plus proche de l'extrémité distale s'étend en oblique par rapport à l'axe longitudinal.

2. Clou huméral suivant la revendication 1, **caractérisé en ce que** l'axe du trou transversal proximal (30) forme avec l'axe longitudinal un angle d'environ 80 DEG.

3. Clou huméral suivant l'une des revendications 1 et 2, **caractérisé en ce que** l'axe du trou transversal distal (36, 36a) dans la section proximale (16, 16a) s'étend également en oblique par rapport à l'axe longitudinal, de telle sorte que les axes du trou transversal proximal et du trou transversal distal (30, 30a et/ou 36, 36a) sont convergents.

4. Clou huméral suivant la revendication 3, **caractérisé en ce que** l'axe du trou transversal distal (36, 36a) forme un angle d'approximativement 75 DEG par rapport à l'axe longitudinal.

5. Clou huméral suivant la revendication 4, **caractérisé en ce que** le déport angulaire du trou transversal distal (36, 36a) par rapport au trou transversal proximal (30, 30a) atteint approximativement 25 DEG.

6. Clou huméral suivant l'une des revendications 3 à 5, **caractérisé en ce que** quatre trous transversaux (30 à 36 et/ou 30a à 36a) sont prévus dans la section proximale (16, 16a) de la tige du clou.

7. Clou huméral suivant la revendication 6, **caractérisé en ce que** le deuxième trou transversal (32, 32a) suivant le trou transversal proximal (30, 30a), est approxi-mativement perpendiculaire par son axe à l'axe longitudinal.

8. Clou huméral suivant la revendication 6, **caractérisé en ce que** le troisième trou transversal (34, 34a) suivant le trou transversal proximal (30, 30a), est approxi-mativement perpendiculaire par son axe à l'axe longitudinal.

9. Clou huméral suivant l'une des revendications 6 à 8, **caractérisé en ce que** le déport angulaire du deuxième trou transversal (32, 32a) par rapport au trou transversal proximal (30, 30a) atteint approximativement 25 DEG.

10. Clou huméral suivant l'une des revendications 6 à 9, **caractérisé en ce que** le déport angulaire du troisième trou transversal (34) par rapport au trou transversal proximal (30) atteint approximativement 90 DEG.

11. Clou huméral suivant l'une des revendications 5 et 9, **caractérisé en ce que** le déport angulaire du deuxième et du quatrième trou transversal (32, 32a, 36, 36a) est dirigé en sens contraire par rapport à l'axe du trou transversal proximal (30, 30a).

12. Clou huméral suivant l'une des revendications 1 à 11, **caractérisé en ce que** la tige du clou a une réalisation massive.

13. Clou huméral suivant l'une des revendications 1 à 12, **caractérisé en ce que** le trou transversal distal (44) est formé dans la zone distale sous forme de trou oblong parallèle à l'axe.

14. Clou huméral suivant l'une des revendications 1 à 12, **caractérisé en ce qu'**une tige du clou est prévue pour l'humérus droit et une tige du clou pour l'humérus gauche et les tiges ont une configuration rectiligne, les trous transversaux dans la section proximale sont agencés différemment pour l'humérus gauche et pour l'humérus droit de telle sorte que, exception faite du trou transversal proximal (30, 30a), l'agencement des trous transversaux (32 à 36 et/ou 32a à 36a) pour la tige de clou gauche est reflété par rapport à l'agencement pour les trous transversaux de la tige de clou droite.

15. Clou huméral suivant l'une des revendications 1 à 14, **caractérisé en ce que** les trous transversaux présentent un taraudage qui correspond au filetage de vis de verrouillage.
